# NEUE EUROPÄISCHE PATENTSCHRIFT

(11) **EP 2 668 963 B2**
(45) Veröffentlichungstag und Bekanntmachung des Hinweises auf die Entscheidung über den Einspruch: **23.01.2019**
(45) Hinweis auf die Patenterteilung: 06.05.2015
(21) Anmeldenummer: 13170224.3
(22) Anmeldetag: 03.06.2013
(51) Int. Cl.: A61L 2/08

(54) **Vorrichtung zum Sterilisieren von Kunststoffbehältnissen mit Sterilisationsüberprüfung**
Device for sterilising polymeric containers with sterilisation checking
Dispositif de stérilisation de récipients polymerique avec contrôle de la stérilisation

(30) Priorität: 01.06.2012 DE 102012104753
(43) Veröffentlichungstag der Anmeldung: 04.12.2013
(73) Patentinhaber: Krones AG, 93073 Neutraubling (DE)
(72) Erfinder: Krüger, Jochen, 93073 Neutraubling (DE)
(74) Vertreter: Bittner, Bernhard

(56) Entgegenhaltungen:
- EP-A1- 0 579 055
- EP-A1- 0 609 709
- EP-A1- 0 647 847
- EP-A1- 0 722 789
- WO-A1-2011/047293
- JP-A- 2007 126 171

## Beschreibung

Die vorliegende Erfindung bezieht sich auf eine Vorrichtung zum Sterilisieren von Behältnissen. Derartige Vorrichtungen sind aus dem Stand der Technik seit langem bekannt. So ist es beispielsweise bekannt, dass Behältnisse, insbesondere Kunststoffbehältnisse, mittels Peressigsäure oder Wasserstoffperoxid (H₂O₂) sterilisiert werden. In jüngerer Zeit ist man jedoch bestrebt, auf den Einsatz von Chemikalien bei der Sterilisierung zu verzichten bzw. diesen zu reduzieren. Daher sind aus dem Stand der Technik auch zahlreiche Vorrichtungen zum Sterilisieren mittels Strahlung, beispielsweise Elektronenstrahlung, bekannt.

Diese Verfahren erlauben eine zufriedenstellende Sterilisation der Behältnisse, beispielsweise der Innenwandungen der Behältnisse. Allerdings ist es vergleichsweise schwierig, eine derartige Elektronenbestrahlung der Behältnisse bzw. das Sterilisationsergebnis zu überwachen.

Für die Überwachung des Prozesses der Elektronenstrahlsterilisation wird ein zuverlässiger Nachweis gefordert, dass jeder einzelne Behälter, beispielsweise ein Becher, eine Flasche, eine Kunststoffflasche oder auch Vorformlinge, eine ausreichende Dosis an Elektronenenergie bzw. Strahlungsenergie abbekommen hat. So wird beispielsweise gefordert, dass auf der gesamten Oberfläche des Behältnisses lückenlos eine Dosis von 25 kGy appliziert wurde.

Zur Überwachung der zuverlässigen Bestrahlung sind unterschiedliche Ansätze bekannt. So ist es beispielsweise bekannt, den Elektronenstrom zu messen und zu überwachen. Dabei kann beispielsweise der Elektronenstrom eines Emitters mittels einer Metallplatte gemessen werden und in Korrelation mit der Sterilisationswirkung bei diesem Strom gebracht werden. Allerdings kann bei diesem Verfahren nicht während der eigentlichen Sterilisation gemessen werden. Weiterhin ist es auch bekannt, ein Mess- und Schutzgitter eines Elektronenstrahlemitters zu verwenden. Dabei wird statt eines Metallbleches mittels eines Metallgitters ein Teil der Strahlung während der Sterilisation abgefangen und quantitativ bewertet.

Auch ist es beispielsweise aus der US 6,560,714 B2 bekannt, einen Draht zu verwenden, der permanent die Intensität des Elektronenstrahls misst. Diese Vorgehensweisen bringen jedoch den Nachteil mit sich, dass ein Teil des Strahls bzw. der Strahlungsenergie nicht für die Sterilisation zur Verfügung steht.

Weiterhin sind Verfahren bekannt, bei denen die Parameter der Emitter permanent überwacht werden. So wird beispielsweise in der DE 2009 034 646 A1 vorgeschlagen, den Strahlstrom und die Strahlspannung zu überwachen. Weiterhin ist es auch bekannt, die Röntgenstrahlung während der Sterilisation zu messen, beispielsweise die Röntgenstrahlen, die bei dem Auftreffen der Elektronen auf eine Oberfläche entstehen und auf diese Weise die Messsignale mit der Behandlungszeit zu korrelieren und damit auch die applizierte Dosis. In diesem Falle können beispielsweise vorbeibewegte Emitter oder Behälter an einem fest installierten Sensor ein zeitlich moduliertes Signal erzeugen.

Grundsätzlich ist es dabei aus dem Stand der Technik bekannt, dass ein Sensor an einer festen Position gegenüber einem Emitterfenster angeordnet ist, wenn beispielsweise der Emitter fest montiert ist. Dies hat jedoch den Nachteil, dass die Messung nur dann stattfinden kann, wenn kein Behältnis zwischen dem Emitter und dem Sensor steht. Die Messung findet also vor oder nach der Sterilisation statt. Falls der Sensor unmittelbar vor dem Emitterfenster platziert ist, fängt er einen Teil der Strahlung ab, der dann nicht zur Sterilisation genutzt werden kann.

Bei einem weiteren Verfahren ist der Emitter beweglich, beispielsweise an einer festen Position an einem Karussell angebracht. Der Strom wird nur dann gemessen, wenn der Emitter den Sensor passiert. Der Stromsensor kann dabei fest an den Strahleinrichtungen, beispielsweise den Strahlfingern montiert sein. Auch in diesem Fall fängt jedoch der Sensor einen erheblichen Teil der Elektronen ab und bildet Schatten auf der zu sterilisierenden Oberfläche. Weiterhin ergibt sich ein zusätzlicher Aufwand, die Kabel für die Messung zu platzieren, da der Emitter so klein sein muss, dass er durch Behälteröffnungen führbar ist.

Die Überwachung der Emitterparameter (Strom und Spannung) haben daher den Nachteil, dass sie nicht die Qualität der Bestrahlung überwachen können. Beispielsweise könnte der Elektronenstrahl nicht gleichmäßig auf das Emitterfenster treffen, das Strahlprofil kann inhomogen sein, ein Teil der emittierten Elektronen kann im Emittergehäuse abgefangen werden.

Zur Messung der Röntgenstrahlung ist eine Alternative aus der DE 2009 034 646 A1 nämlich ein Strahlkopf mit einem Vakuumgehäuse bekannt. Dabei ist eine Überwachungseinrichtung vorgesehen, durch die wenigstens ein Betriebsparameter des Strahlkopfes erfassbar ist und daraus durch Vergleich mit wenigstens einem Referenzwert ein Ausfallvorhersagewert ableitbar ist. Damit wird auch bei dieser Vorrichtung die Strahleinrichtung selbst überprüft.

In diesem Fall ist also ebenfalls ein Sensorsystem vorgesehen, das relativ zum Emitter fixiert ist.

Der vorliegenden Erfindung liegt daher die Aufgabe zugrunde, eine Sterilisation von Kunststoffbehältnissen, insbesondere eine Sterilisation mittels Strahlung und insbesondere Elektronenstrahlung zu überwachen, ohne dabei den eigentlichen Sterilisationsvorgang zu behindern. Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Behältnissen ist in Anspruch 1 definiert.

Im Gegensatz zum Stand der Technik wird also vorgeschlagen, nicht den eigentlichen Sterilisationsvorgang zu messen bzw. zu überprüfen, sondern gewissermaßen dessen Auswirkungen im Nachhinein. Genauer wird der Effekt der Bestrahlung anhand der in einem Bereich bzw. einer Umgebung des Behältnisses befindlichen Gases und/oder eines - insbesondere dem Behältnis entnommenen - flüssigen Mediums gemessen. Bei dem Bereich des Behältnisses kann es sich sowohl um einen außerhalb des Behältnisses liegenden Bereich als auch um einen innerhalb des Behältnisses liegenden Bereich handeln. Bei dem flüssigen Medium kann es sich beispielsweise um ein Spülmedium handeln, mit dem das Behältnis nach seiner Bestrahlung gespült wurde.

Erfindungsgemäß dient die Analyseeinrichtung zur Analyse hinsichtlich durch die Strahlungseinrichtung aus einer Behälterwandung ausgelöster bzw. freigesetzter Massenteilchen.

Vorteilhaft ist die Überprüfungseinrichtung geeignet, um eine Online-Überwachung des Sterilisationsvorgangs, d.h. insbesondere während des Arbeitsbetriebs und besonders bevorzugt auch während des Sterilisationsvorgangs zu überprüfen. Vorteilhaft handelt es sich bei der Strahlung um ionisierende Strahlung und besonders bevorzugt um Elektronenstrahlung. Daneben könnte jedoch auch Röntgenstrahlung oder UV-Strahlung zum Sterilisieren der Behältnisse verwendet werden. Besonders bevorzugt handelt es sich bei der Strahlung um Elektronenstrahlen (E-Beam). Hierbei werden die zu entkeimenden Oberflächen einem Strahl (insbesondere beschleunigter) Elektronen ausgesetzt.

Durch die Wechselwirkung dieser Elektronen mit Keimen oder deren Sporen werden diese deaktiviert und in ihrer Vermehrungsfähigkeit gestoppt. Mit der erfindungsgemäßen Überprüfungseinrichtung wird die Sicherheit des Prozesses der Elektronensterilisation für jedes einzelne Behältnis nachgewiesen. Vorteilhaft dient damit die Überprüfungseinrichtung zur Analyse wenigstens eines im Bereich jedes einzelnen Behältnisses auftretenden gasförmigen Mediums. Es wird also vorteilhaft der Effekt der Bestrahlung anhand des gasförmigen Mediums bzw. der Luft gemessen.

Bei einer weiteren vorteilhaften Ausführungsform ist die Überprüfungseinrichtung geeignet und vorgesehen, für jedes einzelne Behältnis, welches auch durch die Strahlung sterilisiert wurde, ein im Bereich dieses Behältnisses auftretendes Gas (und/oder eine dem Behältnis entnommene Flüssigkeit) zu analysieren.

Bei dem Behältnis handelt es sich um ein Kunststoffbehältnis. Besonders bevorzugt handelt es sich bei dem Behältnis um eine Flasche oder um einen Kunststoffvorformling (der in eine Flasche umgeformt werden kann). Die Anmelderin hat festgestellt, dass im Rahmen einer Elektronenstrahlbehandlung Stoffe aus dem PET ausgelöst werden. Elektronenstrahlung hat eine gewisse Eindringtiefe in Oberflächen und löst dort unterschiedliche Effekte aus. Je nach dem Material können Polymere vernetzt oder degradiert werden. In jedem Fall erfolgt ein Eintrag von Energie, der eine chemische und/oder physikalische Wechselwirkung auf die Oberfläche hat.

Bei der Bestrahlung mit Elektronen werden beispielsweise im PET verschiedene Stoffe erzeugt und aus dem Polymer ausgelöst, die anschließend die Oberfläche verlassen können. So wurden beispielsweise im Rahmen von Versuchen Behältnisse bestrahlt und anschließend mit Reinstwasser gefüllt. In einer anschließenden Analyse wurden in diesem Wasser dabei unter anderem in geringen Mengen Bisphenol A, Isobutyl-Phtalat, Acetaldehyd und weitere höhere Kohlenwasserstoffe (Octadekan, Tricosan, Tetracosan, Eicosan) gefunden, deren Konzentrationen mit der eingestrahlten Strahlendosis korrelieren. Bei einer weiteren Vorgehensweise wäre es auch möglich, nach dem Sterilisationsvorgang das Behältnis innen zu Spülen und das (insbesondere flüssige) Spülmedium anschließend zu analysieren. Bei diesem Verfahren erfolgt damit der Nachweis über eine erfolgreiche Bestrahlung durch eine (insbesondere kontinuierliche) Flüssigkeits- bzw. Wasseranalyse. Diese Analyse findet damit bevorzugt auch inline bzw. während des Arbeitsbetriebs der Anlage statt.

Diesen Effekt macht sich die Erfindung zunutze, indem sie das Gas bzw. die Luft, insbesondere in dem Bereich des Behältnisses, der sterilisiert wurde, misst. Insbesondere handelt es sich bei dem Behältnis um ein PET- Behältnis.

Wie oben erwähnt, sind die bestehenden Konzentrationen der aus dem Material ausgelösten Stoffe sehr gering. Gleichwohl ist es möglich, die Behältnisse nach der Bestrahlung auszublasen. Vor diesem Ausblasen bzw. mit dem Ausblasen können die Stoffe aber mit der Überprüfungseinrichtung gemessen bzw. detektiert werden.

Bei einer bevorzugten Ausführungsform kann die Sterilisierungseinrichtung eine Außenwand oder Innenwand des Behältnisses beaufschlagen. Bevorzugt weisen die Sterilisiereinrichtungen Strahlfinger auf, welche durch die Mündung des Behältnisses in das Innere des Behältnisses eingeführt werden können. Vorteilhaft weisen die Sterilisationseinrichtungen Elektronenbeschleunigungseinrichtungen auf, welche Elektronen beschleunigen. Daneben weisen die Sterilisiereinrichtungen bevorzugt auch Austrittsfenster auf, insbesondere Titanfenster, durch welche Elektronen aus den Sterilisierungseinrichtungen austreten können.

Dabei wäre es möglich, dass sich die Sterilisiereinrichtungen mit den Behältnissen mitbewegen, beispielsweise in Form von Strahlfingern, welche in das Behältnis eintauchen. Daneben wäre es jedoch auch möglich, dass die Sterilisiereinrichtungen stationär angeordnet sind. Daneben können auch mehrere Sterilisationseinrichtungen vorgesehen sein, welche sowohl eine Aussensterilisation als auch eine Innensterilisation der Behältnisse erlauben.

Vorteilhaft weist die Vorrichtung einen Reinraum auf, innerhalb dessen die Behältnisse sterilisiert werden und/oder die Sterilisierung überprüft wird. Vorteilhaft erstreckt sich dieser Reinraum entlang wenigstens eines Abschnittes des Transportpfades der Behältnisse. Vorteilhaft erstreckt sich der Reinraum sowohl über den Bereich des Sterilisierens als auch den Bereich des Überprüfens der Sterilisation.

Bei einer weiteren vorteilhaften Ausführungsform weist die Vorrichtung eine Temperaturmesseinrichtung zum Messen einer Innentemperatur innerhalb der sterilisierten Behältnisse und/oder einer Temperatur der direkten Umgebung der Behältnisse auf. Auch diese Temperatur kann dabei einen Effekt auf die Bewertung des analysierten Gases haben.

Bei einer weiteren vorteilhaften Ausführungsform weist die Überprüfungseinrichtung ein Aufnahmeelement bzw. eine Sonde zum Aufnehmen wenigstens eines Teils des gasförmigen und/oder flüssigen Mediums auf. Dabei könnte ein sogenannter "Schnüffler" zum Einsatz kommen, wie er aus der Inspektionstechnik bekannt ist. Diese Vorrichtungen saugen eine gewisse Menge des gasförmigen Mediums ein, um dieses zu analysieren. Dabei kann die Messung über ein Ansaugrohr erfolgen, welches Luft aus der Umgebung des Behältnisses oder dem Inneren des Behältnisses ansaugt und an die eigentliche Überprüfungseinrichtung leitet.

Die Überprüfungseinrichtung kann entsprechend angelernt werden, sodass die gemessenen Konzentrationen einzelner Moleküle der applizierten Dosis zugeordnet werden können. Zusätzlich kann mithilfe einer Ausblaseinrichtung ein Luftstrom durch die Behältnisse geschickt werden, der die Stoffe auslöst und dem oben erwähnten "Schnüffler" zuführt.

Bei einer weiteren vorteilhaften Ausführungsform weist die Vorrichtung eine Ausschleuseinrichtung zum Ausschleusen von Behältnissen aus dem Transportpfad auf. Vorteilhaft kann dabei eine Ausschleuseinrichtung diejenigen Behältnisse ausschleusen, welche von der Überprüfungseinrichtung als nicht oder unzureichend sterilisiert erkannt wurden. Vorteilhaft ist dabei diese Ausschleuseinrichtung entlang des Transportpfads nach der Überprüfungseinrichtung angeordnet.

Bei einer weiteren vorteilhaften Ausführungsform weist die Überprüfungseinrichtung ein Massenspektrometer auf. Mithilfe dieses Massenspektrometers können bevorzugt einzelne Bestandteile der aufgenommenen Luft bzw. des aufgenommenen Gases analysiert werden und auf diese Weise auf die Effizienz des Sterilisationsvorgangs rückgeschlossen werden. Allgemein ist die Überprüfungseinrichtung geeignet, um Komponenten des zu analysierenden Gases zu erkennen und/oder hinsichtlich ihres mengenmäßigen Anteils zu bestimmen. Neben oder anstelle eines Massenspektrometers kommen jedoch auch andere Überprüfungseinrichtungen in Betracht wie etwa optische Überwachungseinrichtungen.

Da die Menge der ausgelösten Stoffe im direkten Zusammenhang mit der applizierten Dosis steht, kann so sicher festgestellt werden, ob das jeweilige Behältnis eine ausreichende Dosis erhalten hat. Damit sollen genau die Stoffe, die durch die Elektronenbestrahlung ausgelöst werden, bei der Behältersterilisation als Nachweis dienen, eben dass sie ausdiffundiert sind und dass der Prozess damit in ausreichendem Maße stattgefunden hat.

Bei einer weiteren vorteilhaften Ausführungsform ist die Überprüfungseinrichtung stationär angeordnet. So kann die Überprüfungseinrichtung beispielsweise geringfügig oberhalb der Behältnisse angeordnet sein und insbesondere einen Bereich um die Mündung bzw. das darin auftretende gasförmige Medium untersuchen.

Vorteilhaft weist die Überprüfungseinrichtung ein Ansaugelement zum Ansaugen wenigstens eines vorgegebenen Anteils des gasförmigen Mediums auf. Dieses durch das Ansaugelement angesaugte gasförmige Medium wird entsprechend analysiert.

Bei einer weiteren vorteilhaften Ausführungsform weist die Überprüfungseinrichtung eine Steuerungseinrichtung auf, welche bewirkt, dass wenigstens teilweise ein gasförmiges Medium angesaugt wird und/oder dass wenigstens zeitweise ein gasförmiges Medium ausgestoßen wird. So wäre es möglich, zunächst das gasförmige Medium anzusaugen, diese zu analysieren und anschließend - vorteilhaft wieder vollständig - auszustoßen, damit eine weitere Analyse (bei einem folgenden Behältnis) stattfinden kann, ohne dass diese durch Reste des für die Analyse des vorgehenden benötigten Gases verfälscht wird.

Vorteilhaft steuert die Steuerungseinrichtung das Ansaugen und/oder Ausstoßen des gasförmigen Mediums in Abhängigkeit von einem Transport der Behältnisse. So kann beispielsweise eine Ansaugung erfolgen, wenn sich ein bestimmtes Behältnis im Bereich der Überprüfungseinrichtung befindet und die Ausstoßung kann insbesondere in einem Zwischenraum zwischen zwei Behältnissen erfolgen. Vorteilhaft transportiert damit die Transporteinrichtung die zu sterilisierenden Behältnisse vereinzelt.

Bei einer weiteren vorteilhaften Ausführungsform ist wenigstens ein Bereich der Überprüfungseinrichtung wenigstens zeitweise an einem Bereich der Mündungen der sterilisierten Behältnisse befindlich. So ist es möglich, dass die Überprüfungseinrichtung beispielsweise oberhalb der transportierten Behältnisse angeordnet ist bzw. seitlich bezüglich den transportierten Behältnissen und die Behältnisse jeweils an der Überprüfungseinrichtung vorbeitransportiert werden.

Bei einer weiteren vorteilhaften Ausführungsform weist die Vorrichtung wenigstens eine Ausblaseinrichtung zum Ausblasen der Behältnisse auf. Dabei ist es möglich, dass diese Ausblaseinrichtung nach der Überprüfungseinrichtung angeordnet ist und nach dem Überprüfungsvorgang das Behältnis durch Ausblasen reinigt. Es wäre jedoch auch möglich, dass die Ausblaseinrichtung derart angeordnet ist, dass gleichzeitig oder nach dem Ausblasen das Überprüfen erfolgt. So kann beispielsweise durch das Ausblasen der Behältnisse gleich das zu analysierende Gas aus dem Behältnis heraus gedrängt werden.

Vorteilhaft ist eine Unterdruckerzeugungseinrichtung und insbesondere eine Pumpe vorgesehen, welche in einem Bereich der Überprüfungseinrichtung einen Unterdruck erzeugt. Bevorzugt ist eine Leitungseinrichtung - insbesondere eine Kapillare - vorgesehen, über welche das zu untersuchende Gas der Überprüfungseinrichtung zugeführt werden kann. Vorteilhaft ist weiterhin eine Entfernungseinrichtung vorgesehen, welche das zu analysierende Gas wieder aus einem Analyseraum abzieht. Bei dieser Entfernungseinrichtung kann es sich beispielsweise um eine Vakuumpumpe handeln.

Daneben weist die Überprüfungseinrichtung vorteilhaft eine loniosierungseinrichtung auf, welche das zu analysierende Gas wenigstens teilweise ionisiert. Bei einer weiteren vorteilhaften Ausführungsform weist die Überprüfungseinrichtung ein Quattrupol-Spektrometer auf, welches das zu analysierende Gas nach dessen Gewicht(santeilen) sortiert um so bestimmte Moleküle anhand ihres Gewichts identifizieren. Es findet bevorzugt ein permanenter Gasstrom statt, der durch die Kapillare bzw. die Leitungseinrichtung angesaugt wird. Dieser Gasstrom kann - insbesondere permanent - auf das Vorhandensein einer bestimmten Molekülmasse untersucht werden.

Wenn man die Kapillare nicht zum Schnüffeln in den Behälter einführen kann oder will, kann auch ein definiertes Gas in den Behälter eingeblasen werden und die ausströmende Luft ausserhalb der Behälter auf Beimischungen der relevanten Moleküle untersucht werden.

Dabei kann sich die Ausblaseinrichtung mit den jeweiligen Behältnissen mitbewegen oder auch stationär angeordnet sein.

Die vorliegende Erfindung ist weiterhin auf ein Verfahren zum Sterilisieren von Behältnissen gemäß Anspruch 9 gerichtet.

Vorteilhaft weist die Überprüfungseinrichtung ein Massenspektrometer auf. Damit analysiert vorteilhaft die Überprüfungseinrichtung das auftretende Gas und/oder die aus dem Behältnis stammende Flüssigkeit hinsichtlich unterschiedlicher Eigenschaften dessen Moleküle, beispielsweise hinsichtlich der Massen der auftretenden Moleküle.

Bei einem weiteren vorteilhaften Verfahren gibt die Überprüfungseinrichtung wenigstens einen Wert aus, der für die Analyse des auftretenden Gases charakteristisch ist. Dabei kann es sich beispielsweise um einen oder mehrere Werte handeln, die für auftretende Moleküle des analysierten Gases charakteristisch sind. Vorteilhaft gibt die Überprüfungseinrichtung wenigstens einen Wert aus, der für den Massenanteil eines bestimmten Moleküls charakteristisch ist.

Bei einer weiteren vorteilhaften Ausführungsform weist die Überprüfungseinrichtung wenigstens eine Vergleichseinrichtung auf, welchen wenigstens einen von der Überprüfungseinrichtung ermittelten Wert mit einem gespeicherten Wert vergleicht und besonders bevorzugt in Abhängigkeit mit diesem Vergleich ein Ergebnis oder eine Aussage ausgibt.

Bei einem weiteren vorteilhaften Verfahren erfolgt die Untersuchung im Hinblick auf durch die Strahlung verursachte Fremdstoffe bzw. in Hinblick auf die durch die Strahlung aus dem Kunststoffmaterial der Behältnisse ausgelöste Kunststoffe.

Bei einer weiteren vorteilhaften Ausführungsform analysiert die Überprüfungseinrichtung das gasförmige Medium und/oder das flüssige Medium während einer Bewegung der Behältnisse.

Vorteilhaft ist eine Untersuchungszeit < 100 ms, bevorzugt < 10 ms und bevorzugt < 1 ms und besonders bevorzugt < 0,2 ms. Die tatsächliche Messzeit hängt auch von den gewünschten Messergebnissen ab. In einer Messzeit zwischen 1 und 10 ms können auch schwache Konzentrationen oder mehrere Stoffe gleichzeitig nachgewiesen werden.

Vorteilhaft wird damit die Messung inline, d.h. insbesondere für jedes einzelne Behältnis in der Produktion durchgeführt. Damit erfolgt vorteilhaft eine Inline- Prozessüberwachung für die Sterilisation der Behältnisse.

Bei einem weiteren vorteilhaften Verfahren saugt die Überprüfungseinrichtung wenigstens zeitweise das gasförmige Medium ein. Dieses so eingesaugte Medium wird bevorzugt analysiert.

Bei einem weiteren vorteilhaften Verfahren wird vor und/oder während der Überprüfung des gasförmigen Mediums durch die Überprüfungseinrichtung in die Behältnisse ein gasförmiges Medium eingeblasen. Bei diesem gasförmigen Medium kann es sich beispielsweise um ein gefiltertes Gas, um Reinluft, um ein Inertgas, Edelgas oder auch ein Edelgasgemisch handeln. Dieses Gemisch weist dabei besonders bevorzugt genau bekannte Molekülanteile auf und dient besonders bevorzugt, um das bei der Sterilisation entstehende Gas aus dem Behältnis zu drängen.

Vorteilhaft wird ein Anteil des so aus dem Behältnis heraus gedrängten Gasgemisches untersucht, bzw. analysiert.

Weitere Vorteile und Ausführungsformen ergeben sich aus den beigefügten Zeichnungen:
Darin zeigen:
- Fig. 1: Eine schematische Darstellung einer erfindungsgemäßen Vorrichtung zum Sterilisieren von Behältnissen; und
- Fig. 2: eine Detaildarstellung einer Vorrichtung zum Sterilisieren von Behältnissen.

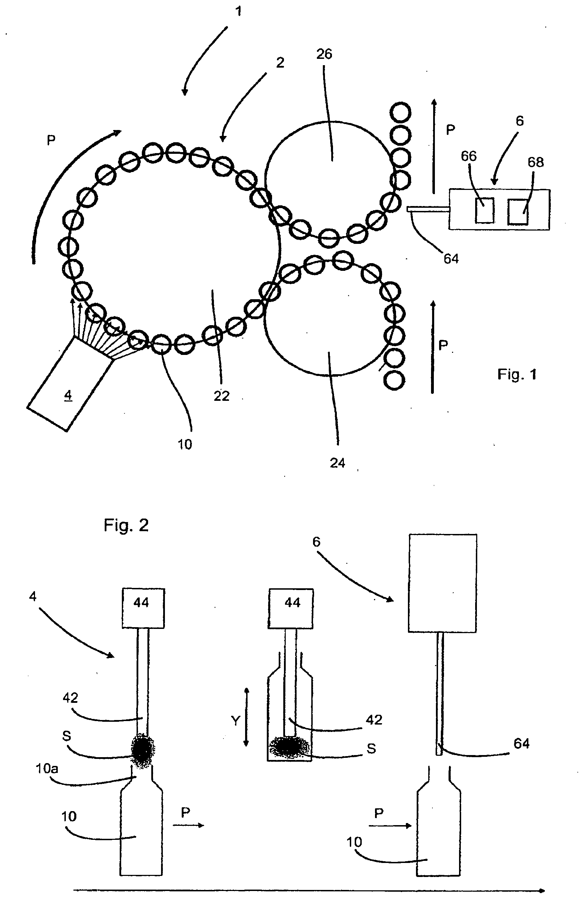

Fig. 1 zeigteine erfindungsgemäße Vorrichtung 1 zum Sterilisieren von Behältnissen. Dabei werden die Behältnisse 10 entlang des Transportpfades P zunächst über eine Zuführeinrichtung, wie ein Zuführrad 24, der Vorrichtung 1 zugeführt und anschließend während ihres Transports mittels einer zweiten Transporteinheit 22 durch eine Sterilisationseinrichtung 4 sterilisiert. Bei der in Fig. 1 gezeigten Ausführungsform erfolgt hier eine Sterilisation der Außenoberflächen der Behältnisse, es kann jedoch mit diesem Abschnitt auch oder alternativ eine Innensterilisation der Behältnisse stattfinden.

Das Bezugszeichen 22 kennzeichnet eine zweite Transporteinheit, wie insbesondere ein Transportrad, an dem die 10 gefördert werden. Es wäre dabei auch möglich, dass zwei oder mehrere Sterilisationseinrichtungen 4 vorgesehen sind, welche an dem Transportrad 22 angeordnet sind und sich damit mit dem Behältnis 10 mitbewegen. Das Bezugszeichen 2 kennzeichnet die Transporteinrichtung in ihrer Gesamtheit.

Das Bezugszeichen 26 kennzeichnet eine weitere Transporteinheit (welche ebenfalls Bestandteil der Transporteinrichtung ist), welche die bereits sterilisierten Behältnisse abtransportiert. Im Bereich dieser weiteren Transporteinheit 26 ist eine Überprüfungseinrichtung 6 angeordnet, welche den Sterilisationsvorgang der Behältnisse 10 überprüft. Diese Überprüfungseinrichtung 6 dient dabei dazu, um ein Gas bzw. Luft im Bereich der Behältnisse 10 zu analysieren. Diese Überprüfungseinrichtung 6 weist dabei eine - insbesondere als Ansaugeinrichtung ausgebildete Sonde 64 auf, mittels der das Gas im Bereich der Behältnisse angesaugt werden kann. Das Bezugszeichen 66 kennzeichnet eine Steuerungseinrichtung zum Steuern der Überprüfungseinheit 6. Diese Steuerungseinrichtung 66 steuert dabei vorteilhaft die Überprüfungseinrichtung 6 auch in Abhängigkeit von dem Transport der Behältnisse 10. So kann beispielsweise in Abhängigkeit einer Relativposition der Behältnisse bzgl. des Ansaugelements 64 jeweils ein Gas angesaugt oder auch wieder ausgestoßen werden.

Das Bezugszeichen 68 kennzeichnet eine Vergleichseinrichtung, welche ein Analyseergebnis des aufgenommenen Gases mit einem vorgegebenen gespeicherten Wert vergleicht. In Abhängigkeit von diesem Vergleich kann an den Benutzer das Ergebnis ausgegeben werden, welches besagt, ob eine korrekte Sterilisation der Behältnisse stattgefunden hat. Damit weist die Überprüfungseinrichtung vorteilhaft eine Speichereinrichtung auf, in welcher vorteilhaft Referenzdaten für das zu analysierende Gas abgespeichert sind. Dabei können unterschiedliche Referenzdaten z.B. für unterschiedliche Behältnisse bzw. Behältnismaterialien gespeichert sein.

Fig. 2 zeigt eine weitere Ausführungsform einer erfindungsgemäßen Vorrichtung. Hier weist die Strahlungseinrichtung 4 ebenfalls wieder einen Elektronenbeschleuniger 44 auf sowie einen Strahlfinger 42, der in das Innere der Behältnisse 10 einführbar ist. Das Bezugszeichen S kennzeichnet die aus diesem Strahlfinger austretenden Strahlen, die wie hier besonders bevorzugt auf eine Innenwandung des Behältnisses treffen. Das Bezugszeichen 10a kennzeichnet eine Mündung des Behältnisses 10, durch welche hindurch der Strahlfinger 42 in das Behältnis 10 eingeführt wird.

Bevorzugt weist damit die Transporteinrichtung 2 auch Halteelemente auf (nicht gezeigt), welche die Behältnisse halten und in der Richtung Y anheben können, damit so der Strahlfinger 42 in das Innere der Behältnisse 10 eintaucht.

In der Transportrichtung P stromabwärts bezüglich der Strahlungseinrichtung 4 ist wieder die Überprüfungseinrichtung 6 angeordnet. Dabei ist hier das Ansaugelement bzw. eine Sonde 64 oberhalb der Mündungen 10a der Behältnisse 10 angeordnet und nimmt somit ein Gas auf, welches sich beispielsweise in dem Behältnis 10 befindet oder aus diesem austritt.

Daneben kann auch eine Ausblaseinrichtung vorgesehen sein, welche das Gas aus dem Behältnis 10 ausbläst.

### Bezugszeichenliste

- 1: Vorrichtung
- 2: Transporteinrichtung in ihrer Gesamtheit
- 4: Sterilisationseinrichtung
- 6: Überprüfungseinrichtung
- 10: Behältnisse
- 10a: Behältnismündung
- 22: zweite Transporteinrichtung
- 24: Zuführrad
- 26: weitere Transporteinheit
- 42: Strahlfinger
- 44: Elektronenbeschleuniger
- 64: Ansaugeinrichtung / Sonde
- 66: Steuerungseinrichtung
- 68: Vergleichseinrichtung

- P: Transportpfad
- S: Strahlen

## Patentansprüche

1. Vorrichtung (1) zum Sterilisieren von Kunststoffbehältnissen (10) mit einer Transporteinrichtung (2), welche die Behältnisse (10) entlang eines vorgegebenen Transportpfades (P) transportiert, mit einer ersten Sterilisationseinrichtung (4), welche wenigstens einen Bereich der Behältnisse (10) mit einer Strahlung beaufschlagt, um diese Behältnisse zu sterilisieren, und mit einer entlang des Transportpfades (P) der Behältnisse (10) stromabwärts bezüglich der ersten Sterilisationseinrichtung (4) angeordneten Überprüfungseinrichtung (6), welche eine Gasanalyseeinrichtung aufweist, zur Überprüfung des Sterilisationsvorgangs,
**dadurch gekennzeichnet, dass**
die Überprüfungseinrichtung (6) zur Analyse wenigstens eines im Bereich der Behältnisse (10) auftretenden gasförmigen Mediums dient, wobei die Überprüfungseinrichtung zur Analyse hinsichtlich durch die Strahlungseinrichtung aus einer Behälterwandung ausgelöster bzw. freigesetzter Masseteilchen dient.

2. Vorrichtung (1) nach Anspruch 1,
**dadurch gekennzeichnet, dass**
die Überprüfungseinrichtung (6) ein Aufnahmeelement zum Aufnehmen wenigstens eines Teils des gasförmigen Mediums aufweist.

3. Vorrichtung (1) nach wenigstens einem der vorangegangenen Ansprüche,
**dadurch gekennzeichnet, dass**
die Überprüfungseinrichtung (6) ein Massenspektrometer aufweist.

4. Vorrichtung (1) nach wenigstens einem der vorangegangenen Ansprüche,
**dadurch gekennzeichnet, dass**
die Überprüfungseinrichtung (6) stationär angeordnet ist.

5. Vorrichtung (1) nach wenigstens einem der vorangegangenen Ansprüche,
**dadurch gekennzeichnet, dass**
die Überprüfungseinrichtung (6) ein Ansaugelement (64) zum Ansaugen wenigstens eines vorgegebenen Anteils des gasförmigen Mediums aufweist.

6. Vorrichtung (1) nach wenigstens einem der vorangegangenen Ansprüche,
**dadurch gekennzeichnet, dass**
die Überprüfungseinrichtung (6) eine Steuerungseinrichtung (66) aufweist, welche bewirkt, dass wenigstens teilweise ein gasförmiges Medium angesaugt wird und/oder dass wenigstens zeitweise ein gasförmiges Medium ausgestoßen wird.

7. Vorrichtung (1) nach wenigstens einem der vorangegangenen Ansprüche,
**dadurch gekennzeichnet, dass**
wenigstens ein Bereich der Überprüfungseinrichtung wenigstens zeitweise in einem Bereich der Mündungen der sterilisierten Behältnisse (10) befindlich ist.

8. Vorrichtung (1) nach wenigstens einem der vorangegangenen Ansprüche,
**dadurch gekennzeichnet, dass**
die Vorrichtung (1) wenigstens eine Ausblaseinrichtung zum Ausblasen der Behältnisse aufweist.

9. Verfahren zum Sterilisieren von Kunststoffbehältnissen (10), wobei die Behältnisse (10) mittels einer Transporteinrichtung (2) entlang eines vorgegebenen Transportpfades (P) transportiert werden und wobei wenigstens ein Bereich der Behältnisse mittels einer Sterilisationseinrichtung (4) bestrahlt wird, und wobei wenigstens ein Teil der Behältnisse (10) nach der Bestrahlung hinsichtlich des Sterilisationsvorgangs (4) mittels einer Überprüfungseinrichtung (6), welche eine Gasanalyseeinrichtung aufweist, überprüft wird, wobei die Überprüfungseinrichtung (6) entlang des Transportpfades (P) stromabwärts bezüglich der Sterilisationseinrichtung (4) angeordnet ist,
**dadurch gekennzeichnet, dass**
die Überprüfungseinrichtung (6) wenigstens einen Teil eines in einem Bereich des Behältnisses (10) befindlichen gasförmigen Mediums hinsichtlich durch die Sterilisationseinrichtung aus einer Behälterwandung ausgelöster bzw. freigesetzter Massenteilchen analysiert.

10. Verfahren nach Anspruch 9,
**dadurch gekennzeichnet, dass**
die Überprüfungseinrichtung (6) das gasförmige Medium während einer Bewegung der Behältnisse analysiert.

11. Verfahren nach wenigstens einem der vorangegangenen Ansprüche 9 oder 10,
**dadurch gekennzeichnet, dass**
die Überprüfungseinrichtung wenigstens zeitweise das gasförmige Medium einsaugt.

12. Verfahren nach wenigstens einem der vorangegangenen Ansprüche 9 - 11,
**dadurch gekennzeichnet, dass**
vor und/oder während der Überprüfung des gasförmigen Mediums durch die Überprüfungseinrichtung in die Behältnisse ein gasförmiges Medium eingeblasen wird.

## Claims

1. An apparatus (1) for the sterilization of plastics material containers (10) with a conveying device (2) which conveys the containers (10) along a pre-set conveying path (P), with a first sterilization device (4) which acts upon at least one area of the containers (10) with radiation in order to sterilize these containers, and with a checking device (6) arranged downstream with respect to the first sterilization device (4) along the conveying path (P) of the containers (10) which has a gas analysis device in order to check the sterilization procedure,
**characterized in that**
the checking device (6) serves for analysing at least one gaseous medium occurring in the region of the containers (10), wherein the checking device serves to analyse mass particles released from a container wall by the radiation device.

2. An apparatus (1) according to claim 1,
**characterized in that**
the checking device (6) has a take-up element for taking up at least part of the gaseous medium.

3. An apparatus (1) according to at least one of the preceding claims,
**characterized in that**
the checking device (6) has a mass spectrometer.

4. An apparatus (1) according to at least one of the preceding claims,
**characterized in that**
the checking device (6) is arranged in a stationary manner.

5. An apparatus (1) according to at least one of the preceding claims,
**characterized in that**
the checking device (6) has a drawing-in element (64) for drawing in at least one pre-set portion of the gaseous medium.

6. An apparatus (1) according to at least one of the preceding claims,
**characterized in that**
the checking device (6) has a control device (66) which causes a gaseous medium to be drawn in at least in part and/or a gaseous medium to be discharged at least for a time.

7. An apparatus (1) according to at least one of the preceding claims,
**characterized in that**
at least one area of the checking device is present at least for a time in an area of the apertures of the sterilized containers (10).

8. An apparatus (1) according to at least one of the preceding claims,
**characterized in that**
the apparatus (1) has at least one blasting device for blowing out the containers.

9. A method of sterilizing plastics material containers (10), wherein the containers (10) are conveyed along a pre-set conveying path (P) by means of a conveying device (2) and wherein at least one area of the containers is irradiated by means of a sterilization device (4), and wherein at least a part of the containers (10) is checked by means of a checking device (6) with respect to the sterilization procedure (4) after the irradiation, which checking device has a gas analysis device, wherein the checking device (6) is arranged downstream with respect to the sterilization device (4) along the conveying path (P),
**characterized in that**
the checking device (6) analyses at least part of a gaseous medium present in an area of the container (10) with respect to mass particles released from a container wall by the sterilisation device.

10. A method according to claim 9,
**characterized in that**
the checking device (6) analyses the gaseous medium during a movement of the containers.

11. A method according to at least one of the preceding claims 9 or 10,
**characterized in that**
the checking device draws the gaseous medium in at least for a time.

12. A method according to at least one of the preceding claims 9 to 11,
**characterized in that**
a gaseous medium is blown into the containers before and/or during the checking of the gaseous medium by the checking device.

## Revendications

1. Installation (1) pour la stérilisation de récipients en matière plastique (10), avec un dispositif de transport (2) qui convoie les récipients (10) le long d'un chemin de transport (P) défini, avec un premier dispositif de stérilisation (4) qui soumet au moins une zone des récipients (10) à un rayonnement pour stériliser lesdits récipients, et avec un dispositif de contrôle (6) situé en aval du premier dispositif de stérilisation (4) le long du chemin de transport (P) des récipients (10), présentant un dispositif d'analyse gazeuse pour le contrôle du processus de stérilisation,
**caractérisée en ce que**
le dispositif de contrôle (6) sert à l'analyse d'au moins un milieu gazeux formé au niveau des récipients (10), le dispositif de contrôle servant à l'analyse de particules de masse d'une paroi de récipient libérées par le dispositif de rayonnement.

2. Installation (1) selon la revendication 1,
**caractérisée en ce que**
le dispositif de contrôle (6) comporte un élément d'absorption pour l'absorption d'au moins une partie du milieu gazeux.

3. Installation (1) selon au moins une des revendications précédentes,
**caractérisée en ce que**
le dispositif de contrôle (6) est pourvu d'un spectromètre de masse.

4. Installation (1) selon au moins une des revendications précédentes,
**caractérisée en ce que**
le dispositif de contrôle (6) est fixe.

5. Installation (1) selon au moins une des revendications précédentes,
**caractérisée en ce que**
le dispositif de contrôle (6) est pourvu d'un élément d'aspiration (64) pour l'aspiration d'au moins une part définie du milieu gazeux.

6. Installation (1) selon au moins une des revendications précédentes,
**caractérisée en ce que**
le dispositif de contrôle (6) est pourvu d'un dispositif de commande (66) qui déclenche l'aspiration au moins partiellement d'un milieu gazeux et/ou le refoulement au moins temporairement d'un milieu gazeux.

7. Installation (1) selon au moins une des revendications précédentes,
**caractérisée en ce qu'**
au moins une zone du dispositif de contrôle est au moins temporairement située dans une zone d'embouchure des récipients (10) stérilisés.

8. Installation (1) selon au moins une des revendications précédentes,
**caractérisée en ce que**
ladite installation (1) est pourvue d'au moins un dispositif de soufflage pour le soufflage des récipients.

9. Procédé de stérilisation de récipients en matière plastique (10), lesdits récipients (10) étant convoyés par un dispositif de transport (2) le long d'un chemin de transport (P) défini et au moins une zone des récipients étant soumise à un rayonnement d'un dispositif de stérilisation (4), et le processus de stérilisation (4) étant contrôlé après irradiation au moyen d'un dispositif de contrôle (6) pour au moins une partie des récipients (10) présentant un dispositif d'analyse gazeuse, le dispositif de contrôle (6) étant situé en aval du dispositif de stérilisation (4) le long du chemin de transport (P),
**caractérisée en ce que**
le dispositif de contrôle (6) analyse au moins une part d'un milieu gazeux se trouvant dans une zone du récipient (10) relative à des particules de masse d'une paroi de récipient libérées par le dispositif de stérilisation.

10. Procédé selon la revendication 9,
**caractérisé en ce que**
le dispositif de contrôle (6) analyse le milieu gazeux pendant un déplacement des récipients.

11. Procédé selon au moins une des revendications précédentes 9 ou 10,
**caractérisé en ce que**
le dispositif de contrôle pompe au moins temporairement le milieu gazeux.

12. Procédé selon au moins une des revendications précédentes 9 à 11,
**caractérisé en ce qu'**
un milieu gazeux est soufflé dans les récipients avant et/ou pendant le contrôle du milieu gazeux par le dispositif de contrôle.
